(19) Europäisches Patentamt
European Patent Office
Office européen des brevets

(11) Veröffentlichungsnummer: **0 110 073**
**B1**

(12) # EUROPÄISCHE PATENTSCHRIFT

(45) Veröffentlichungstag der Patentschrift: **01.07.87**

(51) Int. Cl.⁴: **C 07 D 211/78**, A 61 K 31/445

(21) Anmeldenummer: **83110160.5**

(22) Anmeldetag: **12.10.83**

(54) **Verfahren zur Herstellung von Tetrahydropyridinen.**

(30) Priorität: **23.10.82 DE 3239273**

(43) Veröffentlichungstag der Anmeldung: **13.06.84 Patentblatt 84/24**

(45) Bekanntmachung des Hinweises auf die Patenterteilung: **01.07.87 Patentblatt 87/27**

(84) Benannte Vertragsstaaten: **AT BE CH DE FR GB IT LI NL SE**

(56) Entgegenhaltungen:
**EP-A-0 042 089**
**EP-A-0 044 262**
**DE-A-2 658 804**

(73) Patentinhaber: **BAYER AG, Konzernverwaltung RP Patentabteilung, D-5090 Leverkusen 1 Bayerwerk (DE)**

(72) Erfinder: **Wehinger, Egbert, Dr., Gellertweg 33, D-5600 Wuppertal 1 (DE)**
Erfinder: **Kazda, Stanislav, Dr., Gellertweg 18, D-5600 Wuppertal 1 (DE)**
Erfinder: **Knorr, Andreas, Dr., Pahlkestrasse 15, D-5600 Wuppertal 1 (DE)**

EP 0 110 073 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein Verfahren zur Herstellung von Tetrahydropyridinen.

Durch das erfindungsgemäße Verfahren wird erstmals die Herstellung von Tetrahydropyridinen aus entsprechenden Dihydropyridinen in Ausbeuten, wie sie in den nachfolgenden Beispielen beschrieben sind, ermöglicht. A.P. Phillips, J. Am. Chem. Soc. 71, 4003 (1949) beschreibt Mißerfolge bei der Hydrierung von 4-Aryl-1,4-dihydro-3,5-diestern des Hantzsch-Typs. Auch aus der Publikation von J. Kuthan et al., Ind. Eng. Chem. Prod. Res. Dev. 21, 101ff (1982) wird beschrieben, daß eine partielle katalytische Hydrierung von Dihydropyridinen nur dann erfolgt, wenn lediglich eine Doppelbindung mit einem Akzeptor (substituent-bearing $\pi$-electrons or electron pairs) in Wechselwirkung trifft, d. h. wenn der Dihydropyridinring nur eine Carbonyl- oder Estergruppe als Substituent trägt. Dies wird bestätigt durch K. Tsuda et al. in J. Org. Chem., 21, 800ff (1956). Aus dem Stand der Technik war somit eine Überführung von Dihydropyridinen in entsprechende Tetrahydropyridine durch Disproportionierung oder katalytische Hydrierung nur möglich, wenn der Dihydropyridinring lediglich eine Estergruppierung trägt.

Die Erfindung betrifft somit ein Verfahren zur Herstellung von 1,2,3,4-Tetrahydropyridinen der allgemeinen Formel I

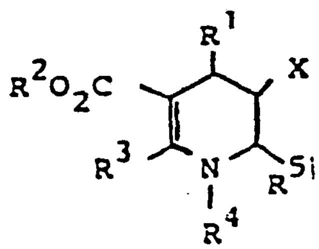

(I)

in welcher

$R^1$ für Phenyl oder Pyridyl steht, wobei der Phenylring gegebenenfalls durch ein oder zwei gleiche oder verschiedene Substituenten aus der Gruppe Fluor, Chlor, Cyano, Trifluormethyl, Trifluormethoxy, Alkyl mit 1 bis 4 C-atomen, Alkoxy mit 1 bis 2 C-Atomen, Tetramethylen oder Phenyl substituiert ist,

$R^2$ für einen geradkettigen, verzweigten oder cyclischen Alkyl- oder Alkenylrest mit bis zu 12 Kohlenstoffatomen steht, der gegebenenfalls durch ein Sauerstoffatom in der Kette unterbrochen ist und/oder der gegebenenfalls substituiert ist durch Fluor, Chlor, Cyano, Hydroxy, Phenyl, Phenoxy oder eine Aminogruppe, die ihrerseits durch zwei gleiche oder verschiedene Substituenten aus der Gruppe Alkyl mit 1 bis 4 C-Atomen oder Benzyl substituiert ist,

$R^3$ und $R^5$ gleich oder verschieden sind und jeweils für Alkyl mit 1 bis 2 C-Atomen stehen,

$R^4$ für Wasserstoff, Alkyl mit 1 bis 4 C-Atomen oder Benzyl steht und

X für die Nitrilgruppe steht oder für den Rest $-CO_2R^6$ steht, wobei $R^6$ die Bedeutung von $R^2$ besitzt und mit $R^2$ gleich oder verschieden ist,

dadurch gekennzeichnet, daß man

a) Dihydropyridinverbindungen der Formel II

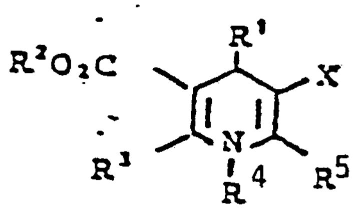

(II)

in der

$R_1$, $R_2$, $R_3$, $R_4$, $R_5$ und X die in Formel (I) angegebene Bedeutung besitzen, oder

b) Pyridinverbindungen der Formel III

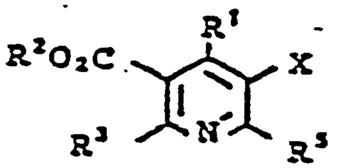

(III)

in der
$R_1$, $R_2$, $R_3$, $R_5$ und X die in Formel (I) angegebene Bedeutung besitzen,
an einer Kathode in Gegenwart eines Elektrolytsystems elektrochemisch reduziert.

Für den Fall, daß $R^4$ in Formel (I) Wasserstoff bedeutet, kann man als Ausgangsmaterial für die elektrochemische Reduktion auch die Pyridinderivate der Formel (III) verwenden,

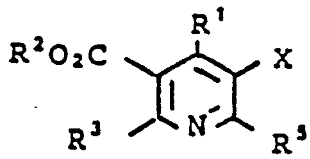

(III)

in welcher die Substituenten
$R^1$, $R^2$, $R^3$, $R^5$ und X die oben angegebene Bedeutung haben.

Die erfindungsgemäß herstellbaren 1,2,3,4-Tetrahydropyridinderivate besitzen wertvolle pharmakologische Eigenschaften. Aufgrund ihrer kreislaufbeeinflussenden Wirkung können sie als antihypertensive Mittel, als periphere und cerebrale Vasodilatatoren sowie als Coronartherapeutika Verwendung finden und sind somit als Bereicherung der Pharmazie anzusehen.

Je nach Art der verwendeten Ausgangsstoffe kann die Darstellung der erfindungsgemäßen Verbindungen durch folgende Formelschemata wiedergegeben werden, wobei der 2,6-Dimethyl-4-phenyl-1,2,3,4-tetrahydropyridin-3,5-dicarbonsäuredimethylester als Beispiel gewählt sei:

A)

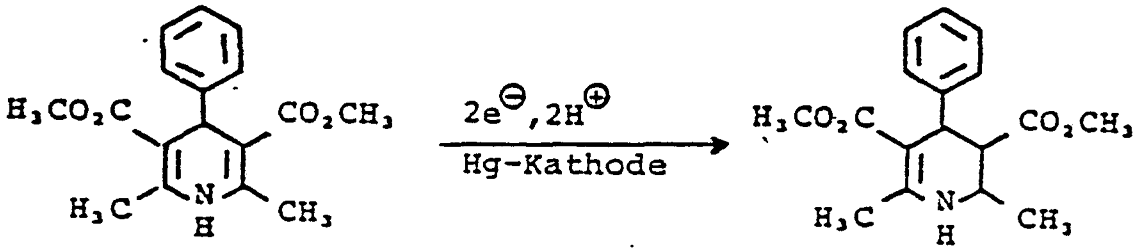


B)

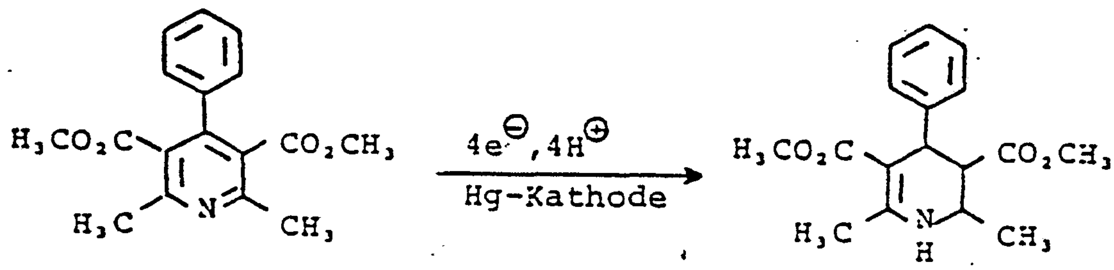


Gemäß Verfahren A wird ein 1,4-Dihydropyridinderivat der allgemeinen Formel (II)

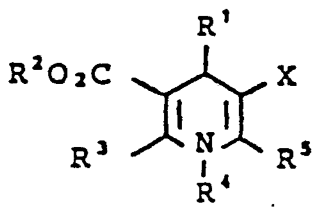

(II)

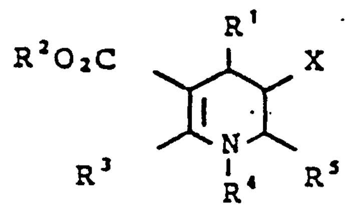

(I)

an einer Quecksilberkathode in einem geeigneten Elektrolytsystem in ein erfindungsgemäßes 1,2,3,4-Tetrahydropyridinderivat der Formel (I) überführt.

Die als Ausgangsstoffe verwendeten Derivate der allgemeinen Formeln (II) und (III) sind literaturbekannt oder können nach literaturbekannten Methoden hergestellt werden (vgl. z. B. U. Eisner und J. Kuthan, Chem. Rev. 72, 1 (1972); E. Wehinger, F. Bossert, G. Franckowiak und H. Meyer, DE-A-2 658 804, veröffentlicht am 6.7.1978).

Zur Durchführung des erfindungsgemäßen Verfahrens wird vorzugsweise eine geteilte Elektrolysezelle mit einer üblichen Kathode, insbesondere mit einem Quecksilberpol als Kathode und einer üblichen Anode, insbesondere einem Platinblech oder Graphit als Anode verwendet (vgl. z. B. N.L. Weinberg, Technique of Electroorganic Synthesis, Part I, John Wiley & Sons (1974)).

Als Reaktionsmedium kommen in erster Linie protische Lösungsmittel wie Wasser, Methanol, Ethanol oder Mischungen derselben in Frage. Darüber hinaus können auch aprotische Lösungsmittel wie Acetonitril oder Dimethylformamid vorteilhaft eingesetzt werden, wenn sie mit Wasser oder einem anderen Protonendonator verdünnt werden.

Als Leitsalze haben sich insbesondere Alkali- oder Tetraalkylammoniumsalze bewährt, wobei vorzugsweise Lithiumtetrafluoroborat, Tetraethylammoniumtetrafluoroborat oder Tetrabutylammoniumchlorid als Beispiele erwähnt seien.

Die Reaktion wird vorzugsweise unter einer Inertgasatmosphäre wie beispielsweise Stickstoff oder einem Edelgas durchgeführt.

Die Reaktionstemperatur kann breit variiert werden, jedoch hat sich der Bereich von 0-50° C, insbesondere 10 bis 30° C als besonders vorteilhaft erwiesen.

Die Reaktion kann sowohl galvanostatisch als auch potentiostatisch durchgeführt werden. Bei potentiostatischer Ausführung wird je nach Substrat bei einem Kathodenpotential von ca. -1,8 bis -2,5 V (gemessen gegen die ges. Kalomelelektrode) so lange elektrolysiert, bis die Stromstärke gegen Null geht oder bis die erforderliche Ladungsmenge (2 Faraday pro Mol 1,4-Dihydropyridin der Formel (II)) durch den Stromkreis geflossen ist. Anschließend wird der Katholyt im Vakuum eingeengt, das Leitsalz durch die üblichen Aufarbeitungsmethoden entfernt und die erfindungsgemäße Substanz gereinigt.

Wenn $R^4$ in Formel (I) Wasserstoff bedeutet, kann man gemäß Verfahrensvariante B als Substrat der elektrochemischen Reduktion auch die Pyridinderivate der allgemeinen Formel (III) verwenden, in welcher die Substituenten $R^1$, $R^2$, $R^3$, $R^5$ und X die oben angegebene Bedeutung haben.

Die Durchführung der Verfahrensvariante B erfolgt ganz analog zu Verfahrensvariante A, lediglich mit dem Unterschied, daß pro Mol Pyridinderivat naturgemäß 4 Faraday-Äquivalente benötigt werden.

Jede Modifikation dieser Verfahren, insbesondere Veränderungen des Elektrolytsystems nach Art, quantitativer Zusammensetzung der Komponenten und pH-Wert sind in gleicher Weise für die Herstellung der nachstehend beschriebenen, erfindungsgemäß hergestellten Verbindungen anwendbar.

Je nach Art der Reste $R^1$ bis $R^5$ und X enthalten diese Verbindungen mindestens 3 Asymmetriezentren und können daher in mehreren stereoisomeren Formen auftreten. Sowohl die Hertsellung der Antipoden als auch der Racemformen als auch der Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z. B. E.L. Eliel, Stereochemistry of Carbon Compounds, McGraw Hill, 1962).

**Herstellungsbeispiele**

**Beispiel 1**

2,6-Dimethyl-4-phenyl-1,2,3,4-tetrahydropyridin-3,5-di-carbonsäuredimethylester

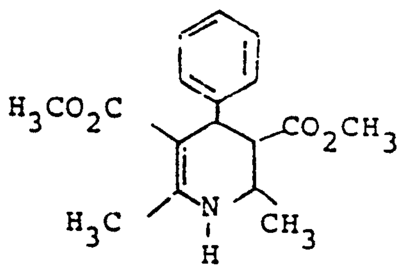

In einer geteilten Elektrolysezelle (s. N.L. Weinberg, Technique of Electroorganic Synthesis, Part I, John Wiley & Sons, (1974)) mit einer Quecksilber-Kathode und einer Platinblech-Anode wurden 5 g (17 mmol) 1,4-Dihydro-2,6-dimethyl-4-phenyl-pyridin-3,5-dicarbon-säuredimethylester in 170 ml einer 0,1 molaren Lösung von Tetraethylammoniumtetrafluoroborat in Methanol bei einem Kathodenpotential von -2,0 V (gegen die gesättigte Kalomelelektrode (SCE)), bei 25°C und unter einer Stickstoffatmosphäre elektrolysiert. Für den Kathoden- und Anodenraum wurde derselbe Grundelektrolyt verwendet.

Nach Durchfluß einer Ladungsmenge von 3300 Coulomb (34 mmol $e^{\ominus}$ ≙ 2 Faraday-Äquivalenten) ging die Stromstärke gegen Null. Der Anolyt wurde verworfen, der Katholyt nach Abtrennung des Quecksilbers im Vakuum eingeengt. Der Rückstand wurde in Dichlormethan aufgenommen, die organische Phase mehrmals mit Wasser gewaschen und nach Trocknen über wasserfreiem Natriumsulfat unter vermindertem Druck abdestilliert. Das zurückbleibende Öl verfestigte sich beim Verreiben mit Ether, es wurde abgesaugt und aus Methanol umkristallisiert.

Schmelzpunkt: 165-167° C, Ausbeute: 2 g (39 %).

**Beispiel 2**

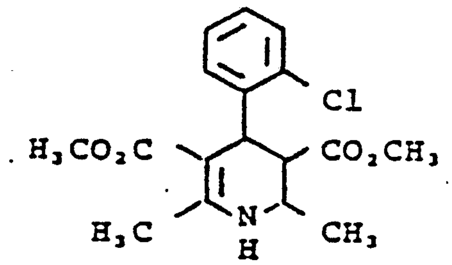

Analog Beispiel 1 wurde durch kathodische Reduktion von 1,4-Dihydro-2,6-dimethyl-4-(2-chlorphenyl)-pyridin-3,5-dicarbonsäuredimethylester in Methanol/Tetraethylammoniumtetrafluoroborat der 2,6-Dimethyl-4-(2-chlorphenyl)-1,2,3,4-tetrahydropyridin-3,5-dicarbonsäuredimethylester vom Fp: 128-130°C erhalten, Ausbeute: 54 %.

**Beispiel 3**

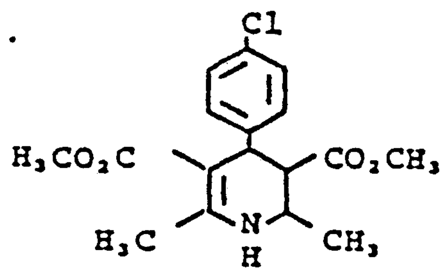

Analog Beispiel 1 wurde durch Reduktion von 1,4-Dihydro-2,6-dimethyl-4-(4-chlorphenyl)-pyridin-3,5-dicarbonsäuredimethylester an der Quecksilberkathode in Methanol/Tetraethylammoniumtetrafluoroborat der 2,6-Dimethyl-4-(4-chlorphenyl)-1,2,3,4-tetrahydropyridin-3,5-dicarbonsäuredimethylester vom Fp: 186-188°C erhalten, Ausbeute: 62 %.

**Beispiel 4**

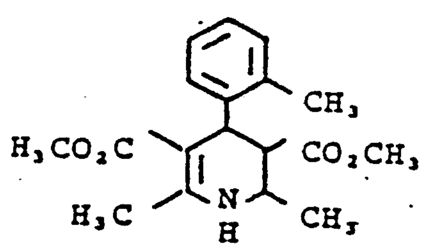

Analog Beispiel 1 wurde durch Reduktion von 1,4-Dihydro-2,6-dimethyl-4-(2-methylphenyl)-pyridin-3,5-dicarbonsäuredimethylester an der Quecksilberkathode in Methanol/Tetraethylammoniumtetrafluoroborat der 2,6-Dimethyl-4-(2-methylphenyl)-1,2,3,4-tetrahydropyridin-3,5-dicarbonsäuredimethylester vom Fp: 125-127°C

erhalten, Ausbeute: 53 %.

**Beispiel 5**

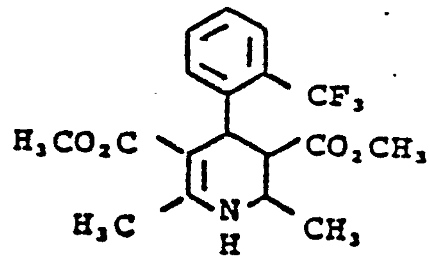

Analog Beispiel 1 wurde durch kathodische Reduktion von 1,4-Dihydro-2,6-dimethyl-4-(2-trifluormethylphenyl)-pyridin-3,5-dicarbonsäuredimethylester an der Quecksilberelektrode in Methanol/Tetraethylammoniumtetrafluoroborat der 2,6-Dimethyl-4-(2-trifluormethylphenyl)-1,2,3,4-tetrahydropyridin-3,5-dicarbonsäuredimethylester vom Fp: 178-181° C erhalten; Ausbeute: 62 % der Theorie.

**Beispiel 6**

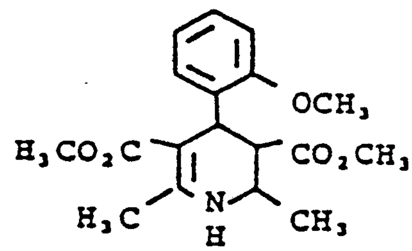

Analog Beispiel 1 wurde durch Reduktion von 1,4-Dihydro-2,6-dimethyl-4-(2-methoxyphenyl)-pyridin-3,5-dicarbonsäuredimethylester an der Quecksilberkathode in Methanol/Tetraethylammoniumtetrafluoroborat der 2,6-Dimethyl-4-(2-methoxyphenyl)-1,2,3,4-tetrahydropyridin-3,5-dicarbonsäuredimethylester vom Fp: 197-199° C erhalten, Ausbeute: 67 %.

**Beispiel 7**

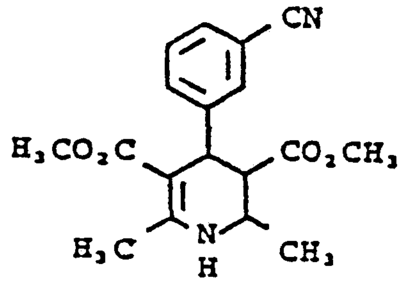

Analog Beispiel 1 wurde durch Reduktion von 1,4-Dihydro-2,6-dimethyl-4-(3-cyanophenyl)-pyridin-3,5-dicarbonsäuredimethylester an der Quecksilberkathode in Methanol/Tetraethylammoniumtetrafluoroborat der 2,6-Dimethyl-4-(3-cyanophenyl)-1,2,3,4-tetrahydropyridin-3,5-dicarbonsäuredimethylester vom Fp: 170-173° C erhalten, Ausbeute: 35 %.

**Beispiel 8**

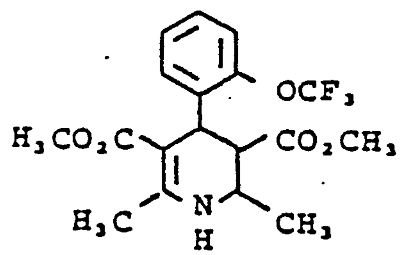

Analog Beispiel 1 wurde durch Reduktion von 1,4-Dihydro-2,6-dimethyl-4-(2-trifluormethoxyphenyl)-pyridin-3,5-di-carbonsäuredimethylester an der Quecksilberkathode in Methanol/Tetraethylammoniumtetrafluoroborat der 2,6-Dimethyl-4-(2-trifluormethoxyphenyl)-1,2,3,4-tetrahydropyridin-3,5-dicarbonsäuredimethylester vom Fp: 145° C erhalten, Ausbeute: 32 %.

**Beispiel 9**

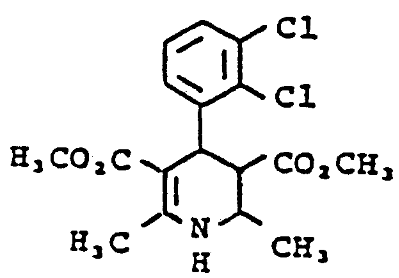

Analog Beispiel 1 wurde durch Reduktion von 1,4-Dihydro-2,6-dimethyl-4-(2,3-dichlorphenyl)-pyridin-3,5-dicarbonsäuredimethylester an der Quecksilberkathode in Methanol/Tetraethylammoniumtetrafluoroborat der 2,6-Dimethyl-4-(2,3-dichlorphenyl)-1,2,3,4-tetrahydropyridin-3,5-dicar-bonsäuredimethylester vom Fp: 170-172° C erhalten, Ausbeute: 64 %.

**Beispiel 10**

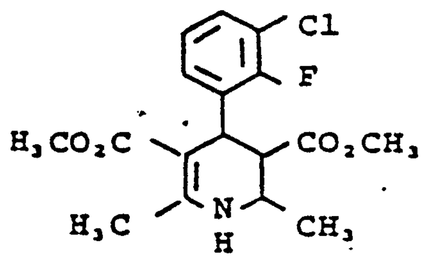

Analog Beispiel 1 wurde durch kathodische Reduktion von 1,4-Dihydro-2,6-dimethyl-4-(2-fluor-3-chlorphenyl)-pyridin-3,5-dicarbonsäuredimethylester an der Quecksilberelektrode in Methanol/Tetraethylammoniumtetrafluoroborat der 2,6-Dimethyl-4-(2-fluor-3-chlorphenyl)-1,2,3,4-tetrahydropyridin-3,5-dicarbonsäuredimethylester vom Fp: 172-174° C erhalten, Ausbeute: 38 %.

**Beispiel 11**

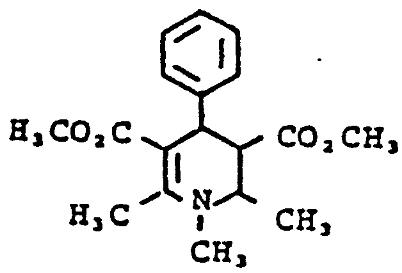

Analog Beispiel 1 wurde durch Reduktion von 1,4-Dihydro-1,2,6-trimethyl-4-phenyl-pyridin-3,5-dicarbonsäuredimethylester an der Quecksilberkathode in Methanol/Tetraethylammoniumtetrafluoroborat der 1,2,6-Trimethyl-4-phenyl-1,2,3,4-tetrahydropyridin-3,5-dicarbonsäuredimethylester vom Fp: 89-90° C erhalten, Ausbeute: 29 %.

**Beispiel 12**

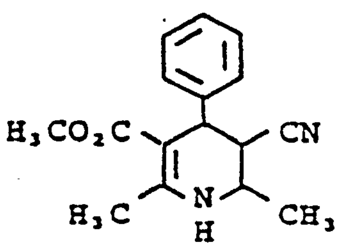

Analog Beispiel 1 wurde durch Reduktion von 3-Cyano-1,4-dihydro-2,6-dimethyl-4-phenyl-pyridin-5-carbonsäuremethylester an der Quecksilberkathode in Methanol/Tetraethylammoniumtetrafluoroborat der 3-Cyano-2,6-dimethyl-4-phenyl-1,2,3,4-tetrahydropyridin-5-carbonsäuremethylester vom Fp: 170-172° C erhalten, Ausbeute: 45 %.

**Beispiel 13**

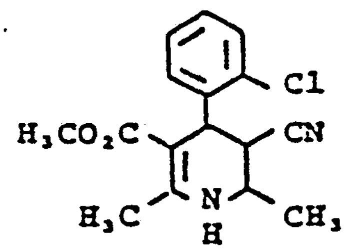

Analog Beispiel 1 wurde durch Reduktion von 3-Cyano-1,4-dihydro-2,6-dimethyl-4-(2-chlorphenyl)-pyridin-5-carbonsäuremethylester an der Quecksilberkathode in Methanol/ Tetrabutylammoniumtetrafluoroborat der 3-Cyano-2,6-dimethyl-4-(2-chlorphenyl)-1,2,3,4-tetrahydropyridin-5-carbonsäuremethylester vom Fp: 168-170° C erhalten, Ausbeute: 71 %.

**Beispiel 14**

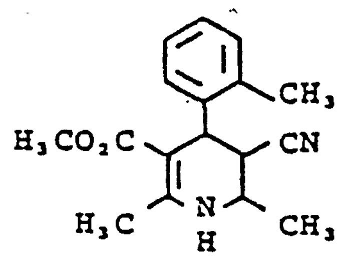

Analog Beispiel 1 wurde durch kathodische Reduktion von 3-Cyano-1,4-dihydro-2,6-dimethyl-4-(2-methylphenyl)-pyridin-5-carbonsäuremethylester an der Quecksilberelektrode in Methanol/Lithiumtetrafluoroborat der 3-Cyano-2,6-dimethyl-4-(2-methylphenyl)-1,2,3,4-tetrahydropyridin-5-carbonsäuremethylester vom Fp: 194-196° C erhalten, Ausbeute: 53 %.

**Beispiel 15**

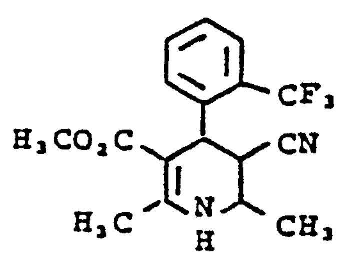

Analog Beispiel 1 wurde durch kathodische Reduktion von 3-Cyano-1,4-dihydro-2,6-dimethyl-4-(2-trifluormethylphenyl)-pyridin-5-carbonsäuremethylester an der Quecksilberelektrode in wäßrigem Ethanol/Tetraethylammoniumtetrafluoroborat der 3-Cyano-2,6-dimethyl-4-(2-trifluormethylphenyl)-1,2,3,4-tetrahydropyridin-5-carbonsäuremethylester vom Fp: 202-204° C erhalten, Ausbeute: 59 %.

**Beispiel 16**

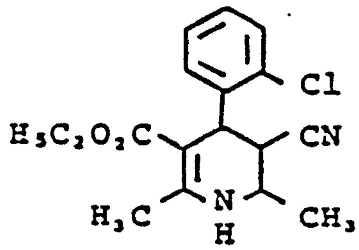

Analog Beispiel 1 wurde durch kathodische Reduktion von 3-Cyano-1,4-dihydro-2,6-dimethyl-4-(2-chlorphenyl)-pyridin-5-carbonsäureethylester in Ethanol/Tetraethylammoniumtetrafluoroborat der 3-Cyano-2,6-dimethyl-4-(2-chlorphenyl)-1,2,3,4-tetrahydropyridin-5-carbonsäureethylester vom Fp: 184-186°C erhalten, Ausbeute: 60 %.

**Beispiel 17**

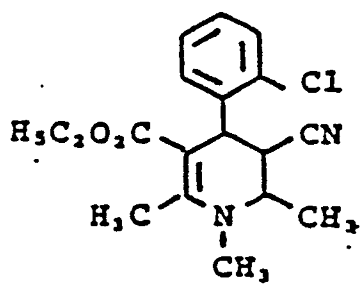

Analog Beispiel 1 wurde durch kathodische Reduktion von 3-Cyano-1,4-dihydro-1,2,6-trimethyl-4-(2-chlorohenyl)-pyridin-5-carbonsäureethylester an der Quecksilberelektrode in Methanol/Tetraethylammoniumtetrafluoroborat der 3-Cyano-1,2,6-trimethyl-4-(2-chlorphenyl)-1,2,3,4-tetrahydropyridin-5-carbonsäureethylester vom Fp: 147-148°C erhalten, Ausbeute: 30 %.

**Beispiel 18**

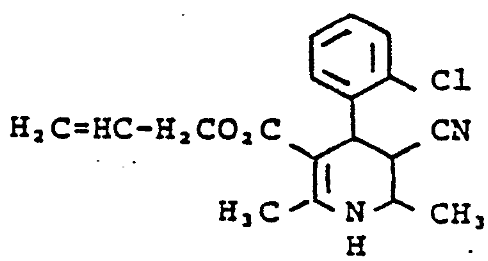

Analog Beispiel 1 wurde durch Reduktion von 3-Cyano-1,4-dihydro-2,6-dimethyl-4-(2-chlorphenyl)-pyridin-5-carbonsäureallylester an der Quecksilberelektrode in Methanol/Tetraethylanmoniumtetrafluoroborat der 3-Cyano-2,6-dimethyl-4-(2-chlorphenyl)-1,2,3,4-tetrahydropyridin-5-carbonsäureallylester vom Fp: 158-159°C

erhalten, Ausbeute: 35 %.

**Beispiel 19**

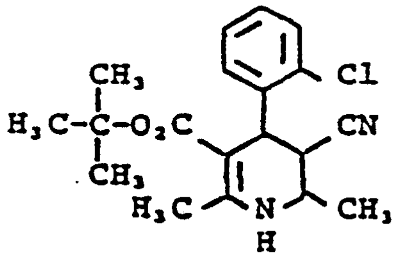

Analog Beispiel 1 wurde durch Reduktion von 3-Cyano-1,4-dihydro-2,6-dimethyl-4-(2-chlorphenyl)-pyridin-5-carbonsäure-tert.-butylester an der Quecksilberkathode in Methanol/Tetraethylammoniumtetrafluoroborat der 3-Cyano-2,6-dimethyl-4-(2-chlorphenyl)-1,2,3,4-tetrahydropyridin-5-carbonsäure-tert.-butylester vom Fp: 204-205° C erhalten, Ausbeute: 32 %.

**Beispiel 20**

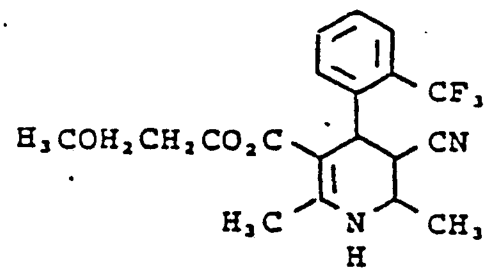

Analog Beispiel 1 wurde durch Reduktion von 3-Cyano-1,4-dihydro-2,6-dimethyl-4-(2-trifluormethylphenyl)-pyridin-5-carbonsäure-(2-methoxyethyl)-ester an der Quecksilberkathode in einem 1:1 Gemisch von Methanol/Acetonitril/ Tetraethylammoniumtetrafluoroborat der 3-Cyano-2,6-di-methyl-4-(2-trifluormethylphenyl)-1,2,3,4-tetrahydropyridin-5-carbonsäure-(2-methoxyethyl)-ester vom Fp.: 182-183°C erhalten, Ausbeute: 54 %.

**Beispiel 21**

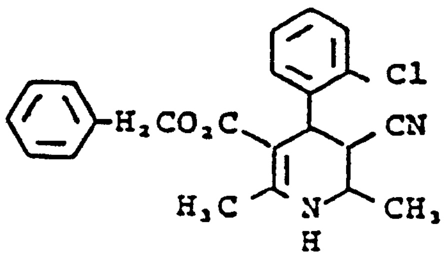

Analog Beispiel 1 wurde durch kathodische Reduktion von 3-Cyano-1,4-dihydro-2,6-dimethyl-4-(2-chlorphenyl)-pyridin-5-carbonsäurebenzylester an der Quecksilberelektrode in Methanol/Tetraethylammoniumtetrafluoroborat der 3-Cyano-2,6-dimethyl-4-(2-chlorphenyl)-1,2,3,4-tetrahydropyridin-5-carbonsäurebenzylester vom Fp: 194-196° C erhalten, Ausbeute: 45 %.

**Beispiel 22**

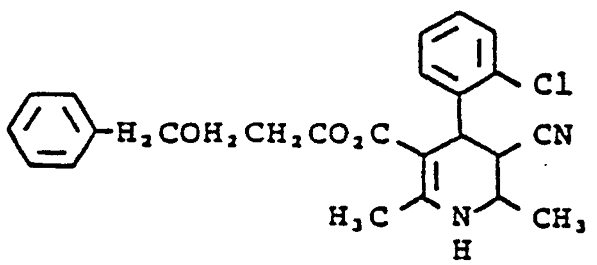

Analog Beispiel 1 wurde durch kathodische Reduktion von 3-Cyano-1,4-dihydro-2,6-dimethyl-4-(2-chlorphenyl)-pyridin-5-carbonsäure-(2-benzyloxyethyl)-ester an der Quecksilberelektrode in Methanol/Acetonitril/Tetraethylammoniumtetrafluoroborat der 3-Cyano-2,6-dimethyl-4-(2-chlorphenyl)-1,2,3,4-tetrahydropyridin-5-carbonsäure-(2-benzyloxyethyl)-ester vom Fp: 88-90° C erhalten, Ausbeute: 25 %.

**Beispiel 23**

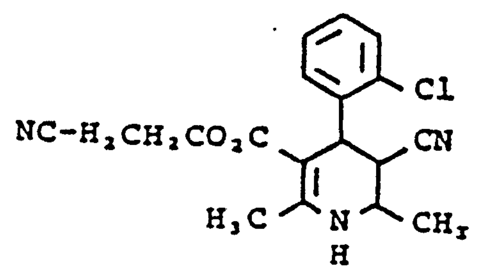

Analog Beispiel 1 wurde durch Reduktion von 3-Cyano-1,4-Dihydro-2,6-dimethyl-4-(2-chlorphenyl)-pyridin-5-carbonsäure-(2-cyanoethyl)-ester an der Quecksilberkathode in Methanol/Tetraethylammoniumtetrafluoroborat der 3-Cyano-2,6-dimethyl-4-(2-chlorphenyl)-1,2,3,4-tetrahydropyridin-5-carbonsäure-(2-cyanoethyl)-ester vom Fp: 189-191° C erhalten, Ausbeute: 25 %.

**Beispiel 24**

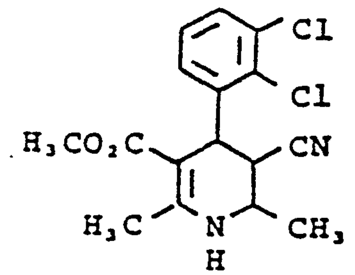

Analog Beispiel 1 wurde durch Reduktion von 3-Cyano-1,4-dihydro-2,6-dimethyl-4-(2,3-dichlorphenyl)-pyridin-5-carbonsäuremethylester an der Quecksilberkathode in Methanol/Tetraethylammoniumtetrafluoroborat der 3-Cyano-2,6-dimethyl-4-(2,3-dichlorphenyl)-1,2,3,4-tetrahydropyridin-5-carbonsäuremethylester vom Fp: 238-240° C erhalten, Ausbeute: 45 %.

**Patentansprüche**

1. Verfahren zur Herstellung von 1,2,3,4-Tetrahydropyridinen der allgemeinen Formel I

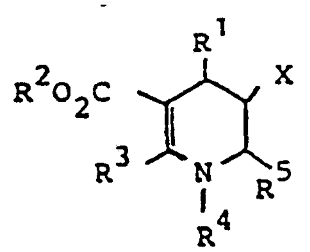

(I)

in welcher

$R^1$ für Phenyl oder Pyridyl steht, wobei der Phenylring gegebenenfalls durch ein oder zwei gleiche oder verschiedene Substituenten aus der Gruppe Fluor, Chlor, Cyano, Trifluormethyl, Trifluormethoxy, Alkyl mit 1 bis 4 C-Atomen, Alkoxy mit 1 bis 2 C-Atomen, Tetramethylen oder Phenyl substituiert ist,

$R^2$ für einen geradkettigen, verzweigten oder cyclischen Alkyl- oder Alkenylrest mit bis zu 12 Kohlenstoffatomen steht, der gegebenenfalls durch ein Sauerstoffatom in der Kette unterbrochen ist und/oder der gegebenenfalls substituiert ist durch Fluor, Chlor, Cyano, Hydroxy, Phenyl, Phenoxy oder eine Aminogruppe, die ihrerseits durch zwei gleiche oder verschiedene Substituenten aus der Gruppe Alkyl mit 1 bis 4 C-Atomen oder Benzyl substituiert ist,

$R^3$ und $R^5$ gleich oder verschieden sind und jeweils für Alkyl mit 1 bis 2 C-Atomen stehen,

$R^4$ für Wasserstoff, Alkyl mit 1 bis 4 C-Atomen oder Benzyl steht und

X für die Nitrilgruppe steht oder für den Rest $-CO_2R^6$ steht, wobei $R^6$ die Bedeutung von $R^2$ besitzt und mit $R^2$ gleich oder verschieden ist,

dadurch gekennzeichnet, daß man gemäß Variante A

a) Dihydropyridinverbindungen der Formel II

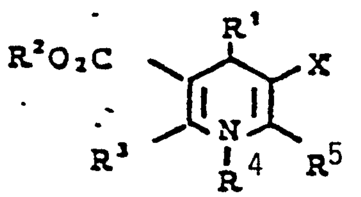

(II)

in der
$R_1$, $R_2$, $R_3$, $R_4$, $R_5$ und X die in Formel (I) angegebene Bedeutung besitzen, oder
gemäß Variante B
b) Pyridinverbindungen der Formel III

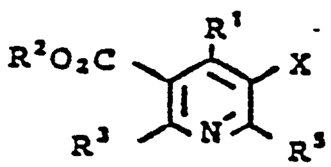

(III)

in der
$R_1$, $R_2$, $R_3$, $R_5$ und X die in Formel (I) angegebene Bedeutung besitzen,
an einer Kathode in Gegenwart eines Elektrolytsystems elektrochemisch reduziert.

2. Verfahren gemäß Anspruch 1, dadurch gekennzeichnet, daß man eine Quecksilberelektrode als Kathode und ein Platinblech oder Graphit als Anode verwendet.

3. Verfahren gemäß den Ansprüchen 1 und 2, dadurch gekennzeichnet, daß man protische Lösungsmittel einsetzt.

4. Verfahren gemäß den Ansprüchen 1 bis 3, dadurch gekennzeichnet, daß man bei einer Temperatur von 0 bis 50°C reduziert.

## Claims

1. Process for the preparation of 1,2,3,4-tetrahydropyridines of the general formula I

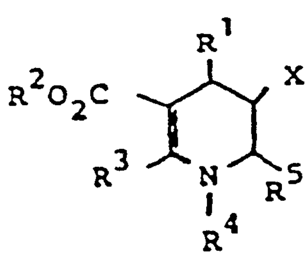

(I)

in which
$R^1$ represents phenyl or pyridyl, the phenyl ring being optionally substituted by one or two identical or different substituents from the group comprising fluorine, chlorine, cyano, trifluoromethyl, trifluoromethoxy, alkyl with 1 to 4 C atoms, alkoxy with 1 to 2 C atoms, tetramethylene and phenyl,

$R^2$ represents a straight-chain, branched or cyclic alkyl or alkenyl radical with up to 12 carbon atoms, which is optionally interrupted by an oxygen atom in the chain and/or which is optionally substituted by fluorine, chlorine, cyano, hydroxyl, phenyl, phenoxy or an amino group, which is in turn substituted by two identical or different substituents from the group comprising alkyl with 1 to 4 C atoms and benzyl,

$R^3$ and $R^5$ are identical or different and each represent alkyl with 1 to 2 C atoms,

$R^4$ represents hydrogen, alkyl with 1 to 4 C atoms or benzyl and

X represents the nitrile group or the radical $-CO_2R^6$, wherein $R^6$ has the meaning of $R^2$ and is identical to or different from $R^2$,

characterised in that, according to variant A

a) dihydropyridine compounds of the formula II

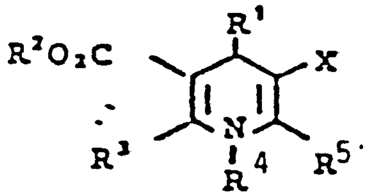

(II)

in which

$R_1$, $R_2$, $R_3$, $R_4$, $R_5$ and X have the meaning given in formula (I) or

according to variant B

b) pyridine compounds of the formula III

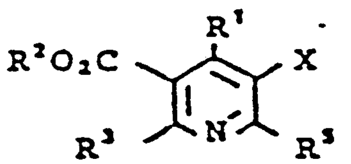

(III)

in which

$R_1$, $R_2$, $R_3$, $R_5$ and X have the meaning given in formula (I)

are electrochemically reduced at a cathode in the presence of an electrolytic system.

2. Process according to Claim 1, characterised in that a mercury electrode is used as the cathode and a platinum plate or graphite is used as the anode.

3. Process according to Claims 1 and 2, characterised in that protic solvents are used.

4. Process according to Claims 1 to 3, characterised in that reduction is carried out at a temperature of 0 to 50°C.

**Revendications**

1. Procédé de préparation de 1,2,3,4-tétrahydro-pyridines de formule générale

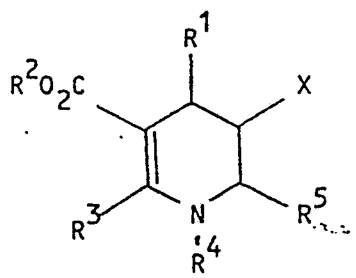

(I)

dans laquelle

$R^1$ représente un groupe phényle ou pyridyle, le noyau phényle portant éventuellement un ou deux substituants identiques ou différents de la classe du fluor, du chlore, des groupes cyano, trifluorométhyle, trifluorométhoxy, alkyle en $C_1$-$C_4$, alcoxy en $C_1$-$C_2$, tétraméthylène ou phényle,

$R^2$ représente un groupe alkyle ou alcényle à chaîne droite ou ramifiée ou cyclique contenant jusqu'à 12 atomes de carbone, éventuellement interrompu dans la chaîne par un atome d'oxygène et/ou éventuellement substitué par le fluor, le chlore, des groupes cyano, hydroxy, phényle, phénoxy ou un groupe amino, lequel peut lui-même porter deux substituants identiques ou différents de la classe des des groupes alkyles en $C_1$-$C_4$

ou benzyle,

$R^3$ et $R^5$, ayant des significations identiques ou différentes, représentent chacun un groupe alkyle en $C_1$-$C_2$,

$R^4$ représente l'hydrogène, un groupe alkyle en $C_1$-$C_4$ ou benzyle, et

X représente le groupe nitrile ou le reste $-CO_2R^6$ dans lequel $R^6$ a la signification de $R^2$ mais est identique ou différent de celui-ci, caractérisé en ce que:

a) selon la variante A, on soumet à réduction électrochimique sur une cathode en présence d'un système d'électrolyte des dihydropyridines de formule II

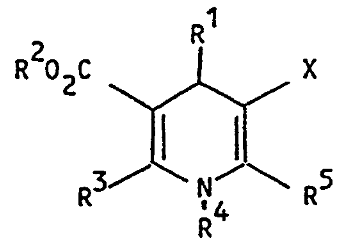

(II)

dans laquelle

$R^1$, $R^2$, $R^3$, $R^4$, $R^5$ et X ont les significations indiquées en référence à la formule I, ou bien

b) selon la variante B, on soumet à réduction électrochimique sur une cathode en présence d'un système d'électrolyte des pyridines de formule III

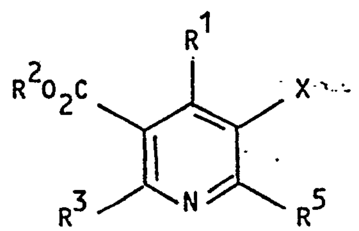

(III)

dans laquelle

$R^1$, $R^2$, $R^3$ $R^4$ et X ont les significations indiquées en référence à la formule I.

2. Procédé selon la revendication 1, caractérisé en ce que l'on utilise une électrode de mercure en tant que cathode et une plaque de platine ou du graphite en tant qu'anode.

3. Procédé selon les revendications 1 et 2, caractérisé en ce que l'on utilise des solvants protoniques.

4. Procédé selon les revendications 1 à 3, caractérisé en ce que l'on réduit à une température de 0 à 50°C.